(19) **Europäisches Patentamt European Patent Office Office européen des brevets**

(11) **EP 4 641 752 A2**

(12) **EUROPEAN PATENT APPLICATION**

(43) Date of publication:
**29.10.2025 Bulletin 2025/44**

(21) Application number: **25171048.9**

(22) Date of filing: **16.04.2025**

(51) International Patent Classification (IPC):
**H01M 10/42** *(2006.01)*

(52) Cooperative Patent Classification (CPC):
**H01M 10/484; H01M 10/4285;** H01M 10/058;
H01M 50/609

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **18.04.2024 KR 20240052137**

(71) Applicants:
• **SAMSUNG SDI CO., LTD.
Yongin-si, Gyeonggi-do 17084 (KR)**
• **Research & Business Foundation
Sungkyunkwan
University
Gyeonggi-do 16419 (KR)**

(72) Inventors:
• **LEE, Il Bok
16678 Suwon-si, Gyeonggi-do (KR)**
• **KIM, Kyoung Ho
16678 Suwon-si, Gyeonggi-do (KR)**
• **KIM, Ji Man
16507 Suwon-si, Gyeonggi-do (KR)**
• **LEE, Jung Ho
01461 Seoul (KR)**

(74) Representative: **Gulde & Partner
Patent- und Rechtsanwaltskanzlei mbB
Berliner Freiheit 2
10785 Berlin (DE)**

(54) **IMPREGNATION EVALUATION DEVICE AND SECONDARY BATTERY PRODUCTION SYSTEM**

(57)    The present disclosure relates to an impregnation evaluation device for evaluating impregnation of an electrode plate by an electrolyte. To this end, an impregnation evaluation device includes a processor configured to calculate a theoretical mass of an electrode plate, and a mass measuring unit configured to measure an actual mass of the electrode plate, wherein the processor is configured to evaluate the impregnation of the electrode plate by comparing the theoretical mass with the actual mass.

# FIG. 5

**Description**

BACKGROUND

**1. Field of the Disclosure**

The present disclosure relates to an impregnation evaluation device for evaluating the impregnation of an electrode assembly and/or a secondary battery production system for manufacturing a secondary battery by evaluating the impregnation and/or impregnating the electrode assembly.

**2. Description of the Related Art**

A secondary battery is a battery which can be charged and discharged, unlike a primary battery which cannot be recharged. Low-capacity secondary batteries are used in portable small electronic devices such as smartphones, feature phones, laptop computers, digital cameras, and camcorders. High-capacity secondary batteries are widely used as power sources for motor driving of hybrid vehicles, electric vehicles, or the like, batteries for power storage, or the like. Secondary batteries include a positive electrode and a negative electrode, an electrode assembly including the electrodes, a case accommodating the electrode assembly, an electrode terminal connected to the electrode assembly.

As technology advances, there is an increasing need for high capacity and/or high output secondary batteries. Accordingly, a plurality of secondary batteries may be used by being electrically connected together. The secondary batteries can be used in electronic devices in the form of a secondary battery module including a plurality of secondary batteries and/or a secondary battery pack including a plurality of secondary battery modules. Such configurations can be used in electronic devices requiring high output and/or high capacity which include, for example, electric vehicles and the like.

Meanwhile, as high capacity and/or high output secondary batteries are required, the importance of an impregnation process for an electrode included in a secondary battery is increasing. The impregnation process of the electrode includes factors such as the sufficiency of the impregnation with an electrolyte and an impregnation speed of the electrode (indicating how quickly the electrode is impregnated by the electrolyte). When the electrode is not sufficiently impregnated with the electrolyte, lithium ions cannot move smoothly to the electrode and a current from the battery decreases. In addition, when the impregnation speed of the electrode is low, there is a problem that the productivity of the secondary battery is lowered.

Therefore, it is important to provide an environment where the electrode can be quickly and sufficiently impregnated with the electrolyte to ensure that the impregnation process of the battery is efficiently carried out.

The information disclosed in this section is only intended to provide a better understanding of the background of the present disclosure, and therefore information that does not constitute the related art may be included.

SUMMARY OF THE DISCLOSURE

The present disclosure is directed to providing an impregnation evaluation device for evaluating the impregnation of a battery.

The present disclosure is also directed to providing an impregnation evaluation device for ensuring a minimum impregnation time for a battery.

The present disclosure is also directed to providing an impregnation evaluation device for providing an environment in which an impregnation process for a battery can be carried out efficiently.

The present disclosure is also directed to providing a secondary battery production system for impregnating an electrode in an appropriate environment.

For example, the present disclosure is directed to providing a secondary battery production system for manufacturing a secondary battery that includes an electrode impregnated in the appropriate environment and having improved impregnation and/or impregnation speed.

However, the technical objects to be solved by the present disclosure are not limited to the above-described objects, and other objects not mentioned herein will be clearly understood by those skilled in the art from the following description.

According to an aspect of the present disclosure, there is provided an impregnation evaluation device, which includes a processor configured to calculate a theoretical mass of an electrode plate, and a mass measuring unit configured to measure an actual mass of the electrode plate, wherein the processor compares the theoretical mass with the actual mass and evaluates the impregnation of the electrode plate.

According to another aspect of the present disclosure, there is provided a secondary battery production system, which includes an impregnation evaluation device configured to compare a theoretical mass of an electrode plate with an actual mass of the electrode plate and evaluate impregnation of the electrode plate, and an impregnation device configured to impregnate the electrode plate based on a result evaluated by the impregnation evaluation device.

BRIEF DESCRIPTION OF THE DRAWINGS

The drawings attached to the present specification illustrate preferred embodiments of the present disclosure and serve to further understanding of the technical spirit of the present disclosure along with the detailed description described below. The present disclosure is not limited to the embodiments described in the drawings.

FIGS. 1 to 4 are cross-sectional views showing a lithium secondary battery according to an embodiment;
FIG. 5 is a block diagram schematically showing an impregnation evaluation device according to an embodiment;
FIG. 6 is a flowchart showing an impregnation evaluation method according to an embodiment;
FIG. 7 is a graph for describing a washing unit according to an embodiment;
FIGS. 8A and 8B are views of an ideal electrode plate without pores and an actual electrode plate with pores;
FIG. 9 is a graph of a theoretical mass versus an actual mass shown according to an embodiment; and
FIG. 10 is a block diagram schematically showing a secondary battery production system according to an embodiment.

DETAILED DESCRIPTION OF EXEMPLARY EMBODIMENTS

Hereinafter, embodiments of the present disclosure will be described in detail. However, these embodiments are presented as examples, the present disclosure is not limited thereto.
Unless otherwise stated herein, when a part such as a layer, a membrane, a region, or a plate is described as being "on" another part, this includes not only a case where the part is "directly above" the other part, but also a case where there are other parts therebetween.
Unless otherwise stated herein, a singular may also include a plural. In addition, unless otherwise stated, "A or B" may mean "including A, including B, or including A and B."
As used herein, "a combination thereof" may mean a mixture, a laminate, a composite, a copolymer, an alloy, a blend, a reaction product of constituents, or the like.
Unless otherwise defined herein, a particle diameter may be an average particle diameter. In addition, the particle diameter is an average particle diameter D50 which refers to diameters of particles whose cumulative volumes are 50 volume% in a particle size distribution. The average particle diameter D50 may be measured by methods well known to those skilled in the art, for example, a particle diameter analyzer, a transmission electron microscope, or a scanning electron microscope. As another method, the average particle diameter D50 value may be obtained by measuring the particle diameters using a measurement device using dynamic light-scattering, conducting data analysis, counting the number of particles in each particle diameter range, and then calculating the average particle diameter therefrom. Alternatively or additionally, the average particle diameter may be measured using a laser diffraction method. When measuring the average particle diameter by the laser diffraction method, more specifically, the average particle diameter D50 based on 50% of the particle diameter distribution in the measurement device may be calculated by dispersing the particles to be measured in a dispersing medium, then introducing the particles into a commercially available laser diffraction particle diameter measurement device (e.g., Microtrac's MT 3000), and applying ultrasonic waves of about 28 kHz with a power output of 60 W.

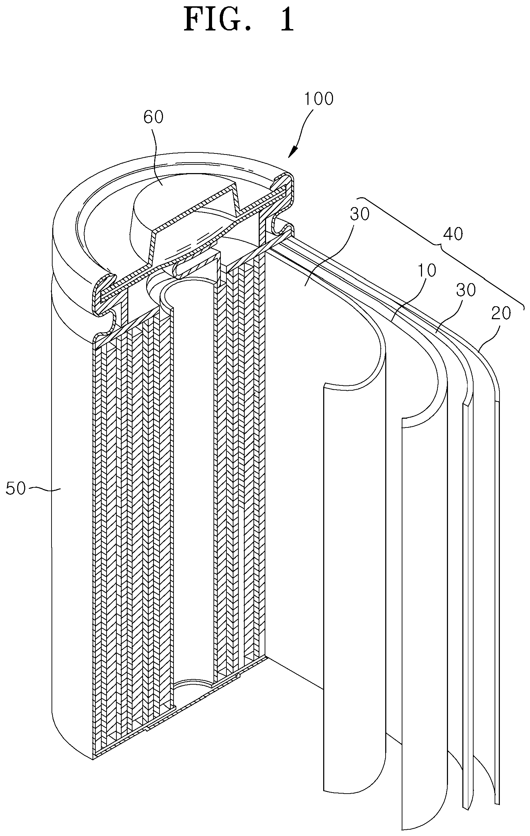

FIGS. 1 to 4 are cross-sectional views showing a lithium secondary battery according to an embodiment.

## Lithium secondary battery 100

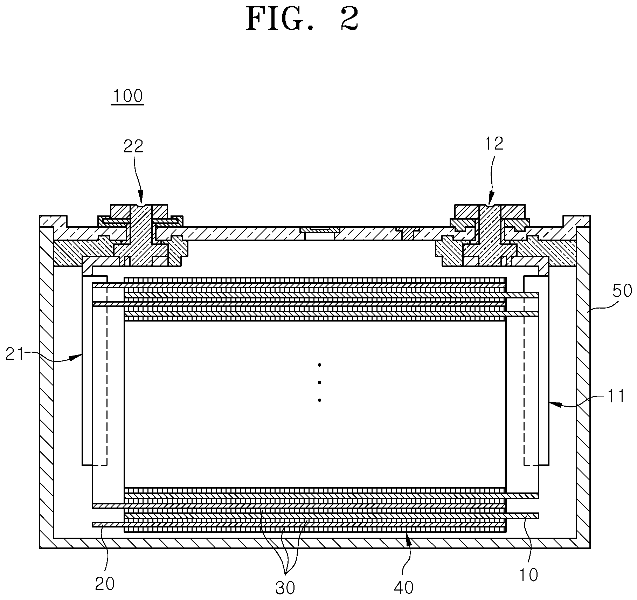

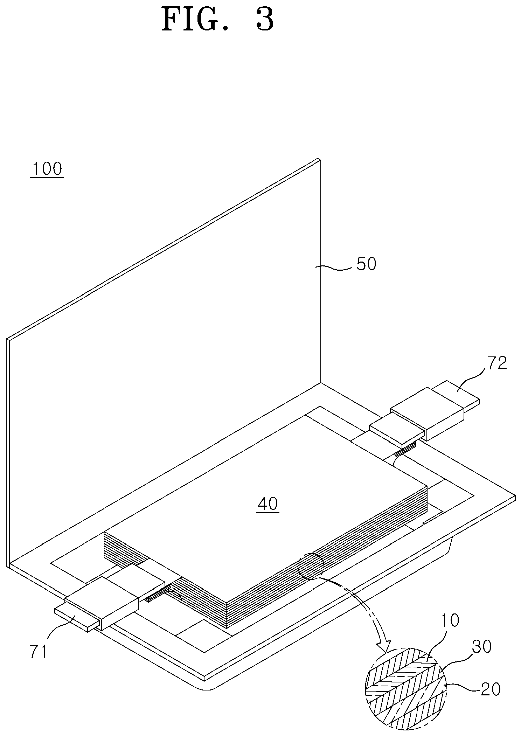

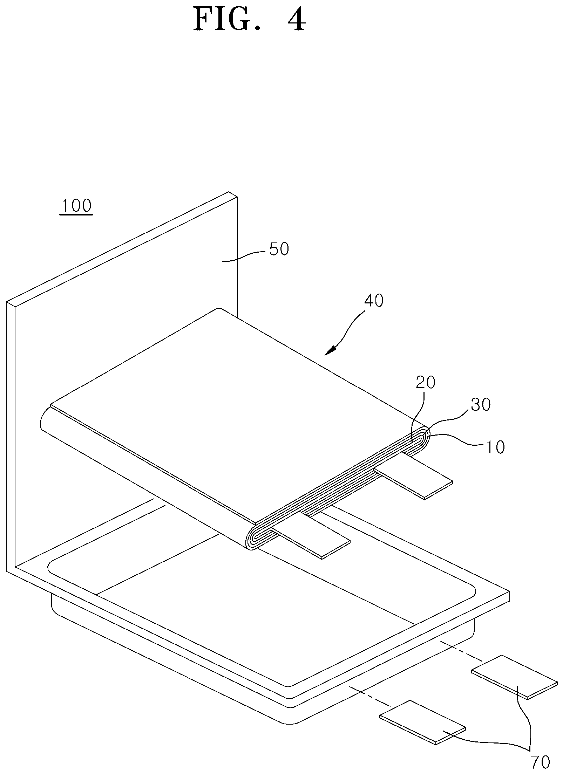

The lithium secondary battery 100 may be classified as a cylindrical shape, a prismatic shape, a pouch shape, a coin shape, etc. depending on its shape. FIGS. 1 to 4 are schematic views showing the lithium secondary battery according to embodiments, where FIG. 1 shows a cylindrical battery, FIG. 2 shows a prismatic battery, and FIGS. 3 and 4 show a pouch-shaped battery. Referring to FIGS. 1 to 4, the lithium secondary battery 100 may include an electrode assembly 40 with a separator 30 interposed between a positive electrode 10 and a negative electrode 20, and a case 50 in which the electrode assembly 40 is accommodated. The positive electrode 10, the negative electrode 20, and the separator 30 may be impregnated with an electrolyte (not shown). The lithium secondary battery 100 may include a sealing member 60 for sealing the case 50 as shown in FIG. 1. In addition, in FIG. 2, the lithium secondary battery 100 may include a positive electrode lead tab 11, a positive electrode terminal 12, a negative electrode lead tab 21, and a negative electrode terminal 22. As shown in FIGS. 3 and 4, the lithium secondary battery 100 may include electrode tabs 70, that is, a positive electrode tab 71 and a negative electrode tab 72, which serve as an electrical passage to guide a current generated from the electrode assembly 40 to outside of the battery.

**Positive electrode active material**

As a positive electrode active material, a compound capable of reversible intercalation and deintercalation of lithium (a lithiated intercalation compound) may be used. Specifically, at least one of composite oxides of a metal selected from cobalt, manganese, nickel, and a combination thereof and lithium may be used as the positive electrode active material.

The composite oxide may be a lithium transition metal composite oxide, and specific examples include a lithium nickel-based oxide, a lithium cobalt-based oxide, a lithium manganese-based oxide, a lithium iron phosphate-based compound, a cobalt-free nickel-manganese-based oxide, or a combination thereof.

As an example, a compound represented by any one of the following chemical formulas may be used: $Li_aA_{1-b}X_bO_{2-c}D_e$ ($0.90 \leq a \leq 1.8$, $0 \leq b \leq 0.5$, $0 \leq c \leq 0.05$); $Li_aMn_{2-b}X_bO_{4-c}D_c$ ($0.90 \leq a \leq 1.8$, $0 \leq b \leq 0.5$, $0 \leq c \leq 0.05$); $Li_aNi_{1-b-c}Co_bX_cO_{2-\alpha}D_\alpha$ ($0.90 \leq a \leq 1.8$, $0 \leq b \leq 0.5$, $0 \leq c \leq 0.5$, $0 < \alpha < 2$); $Li_aNi_{1-b-c}Mn_dX_cO_{2-\alpha}D_\alpha$ ($0.90 \leq a \leq 1.8$, $0 \leq b \leq 0.5$, $0 \leq c \leq 0.5$, $0 < \alpha < 2$); $Li_aNi_bCo_cL^1_{d-}G_eO_2$ ($0.90 \leq a \leq 1.8$, $0 \leq b \leq 0.9$, $0 \leq c \leq 0.5$, $0 \leq d \leq 0.5$, $0 \leq e \leq 0.1$); $Li_aNiG_bO_2$ ($0.90 \leq a \leq 1.8$, $0.001 \leq b \leq 0.1$); $Li_aCoG_bO_2$ ($0.90 \leq a \leq 1.8$, $0.001 \leq b \leq 0.1$); $Li_aMn_{1-b}G_bO_2$ ($0.90 \leq a \leq 1.8$, $0.001 \leq b \leq 0.1$); $Li_aMn_2G_bO_4$ ($0.90 \leq a \leq 1.8$, $0.001 \leq b \leq 0.1$); $Li_aMn_{1-g}G_gPO_4$ ($0.90 \leq a \leq 1.8$, $0 \leq g \leq 0.5$); $Li_{(3-f)}Fe_2(PO_4)_3$ ($0 \leq f \leq 2$); and $Li_aFePO_4$ ($0.90 \leq a \leq 1.8$). In these chemical formulas, A is Ni, Co, Mn, or a combination thereof; X is Al, Ni, Co, Mn, Cr, Fe, Mg, Sr, V, a rare earth element, or a combination thereof; D is O, F, S, P, or a combination thereof; G is Al, Cr, Mn, Fe, Mg, La, Ce, Sr, V, or a combination thereof; and $L^1$ is Mn, Al, or a combination thereof.

As an example, the positive electrode active material may be a high nickel-based positive electrode active material with a nickel content of 80 mol% or more, 85 mol% or more, 90 mol% or more, 91 mol% or more, or 94 mol% or more and 99 mol% or less based on 100 mol% of a metal excluding lithium from the lithium transition metal composite oxide. The high nickel-based positive electrode active material can realize high capacity and can thereby be applied to high capacity, high density lithium secondary batteries.

**Positive electrode 10**

The positive electrode 10 for the lithium secondary battery 100 may include a current collector and a positive electrode active material layer formed on the current collector. The positive electrode active material layer may contain the positive electrode active material and further contain a binder and/or a conductive material. In an example, the positive electrode may contain an additive that may serve as a sacrificial positive electrode.

The amount of the positive electrode active material may range from 90 wt% to 99.5 wt% based on 100 wt% of the positive electrode active material layer. The amount of the binder and the conductive material may each range from 0.5 wt% to 5 wt% based on 100 wt% of the positive electrode active material layer.

The binder may serve to attach the positive electrode active material particles to each other and to also attach the positive electrode active material to the current collector. Representative examples of the binder may include polyvinyl alcohol, carboxymethyl cellulose, hydroxypropyl cellulose, diacetyl cellulose, polyvinyl chloride, carboxylated polyvinyl chloride, polyvinyl fluoride, a polymer containing ethylene oxide, polyvinyl pyrrolidone, polyurethane, polytetrafluoroethylene, polyvinylidene fluoride, polyethylene, polypropylene, styrene-butadiene rubber, (meth)acrylated styrene-butadiene rubber, an epoxy resin, a (meth)acrylic resin, a polyester resin, nylon, etc.. But the present disclosure is not limited to these examples.

The conductive material may be used to impart conductivity to the electrode. In a configured battery, any electronically conductive material that does not cause a chemical change may be used. Examples of the conductive material include a carbon-based material such as natural graphite, artificial graphite, carbon black, acetylene black, Ketjenblack, carbon fibers, carbon nanofibers, carbon nanotubes; a metal-based material containing copper, nickel, aluminum, silver, etc. in the form of a metal powder or metal fiber; a conductive polymer such as a polyphenylene derivative; or a mixture thereof.

Aluminum may be used as the current collector but the present is not limited thereto.

**Negative electrode active material**

The negative electrode active material may include a material capable of reversible intercalation/deintercalation of lithium ions, lithium metal, an alloy of lithium metal, a material capable of doping and dedoping lithium, or a transition metal oxide.

The material capable of reversible intercalation/deintercalation of lithium ions may be a carbon-based negative electrode active material, and may include, for example, crystalline carbon, amorphous carbon, or a combination thereof. Examples of the crystalline carbon include graphite, such as amorphous, plate-like, flake, spherical, or fibrous natural graphite or artificial graphite, and examples of the amorphous carbon include soft carbon, hard carbon, mesophase pitch carbide, calcined coke, etc.

As the alloy of lithium metal, an alloy of lithium and a metal selected from Na, K, Rb, Cs, Fr, Be, Mg, Ca, Sr, Si, Sb, Pb, In, Zn, Ba, Ra, Ge, Al, and Sn may be used.

As the material capable of doping and dedoping lithium, a Si-based negative electrode active material or a Sn-based negative electrode active material may be used. The Si-based negative electrode active material may include silicon, a silicon-carbon composite, $SiO_x$ (0<x<2), a Si-Q alloy (Q is selected from an alkali metal, an alkaline earth metal, a group 13 element, a group 14 element (excluding Si), a group 15 element, a group 16 element, a transition metal, a rare earth element, and a combination thereof), or a combination thereof. The Sn-based negative electrode active material may be Sn, $SnO_2$, a Sn-based alloy, or a combination thereof.

The silicon-carbon composite may be a composite of silicon and amorphous carbon. According to an embodiment, the silicon-carbon composite may be in the form of silicon particles and amorphous carbon coated on surfaces of the silicon particles. For example, the silicon-carbon composite may include a secondary particle (core) in which silicon primary particles are assembled and an amorphous carbon coating layer (shell) located on a surface of the secondary particle. The amorphous carbon may also be located between the silicon primary particles. For example, the silicon primary particles may be coated with the amorphous carbon. The secondary particle may be dispersed in an amorphous carbon matrix.

The silicon-carbon composite may further include crystalline carbon. For example, the silicon-carbon composite may include a core containing crystalline carbon and silicon particles and an amorphous carbon coating layer located on a surface of the core.

The Si-based negative electrode active material or the Sn-based negative electrode active material may be used by being mixed with a carbon-based negative electrode active material.

**Negative electrode 20**

The negative electrode 20 for the lithium secondary battery 100 may include a current collector and a negative electrode active material layer located on the current collector. The negative electrode active material layer may contain a negative electrode active material and may further contain a binder and/or a conductive material.

The negative electrode active material layer may include, for example, 90 wt% to 99 wt% of the negative electrode active material, 0.5 wt% to 5 wt% of the binder, and 0 wt% to 5 wt% of the conductive material.

The binder may serve to attach the negative electrode active material particles and to also attach the negative electrode active material to the current collector. The binder may be a non-aqueous binder, an aqueous binder, a dry binder, or a combination thereof.

The non-aqueous binder may include polyvinyl chloride, carboxylated polyvinyl chloride, polyvinyl fluoride, an ethylene propylene copolymer, polystyrene, polyurethane, polytetrafluoroethylene, polyvinylidene fluoride, polyethylene, polypropylene, polyamide-imide, polyimide, or a combination thereof.

The aqueous binder may be selected from styrene-butadiene rubber, (meth)acrylated styrene-butadiene rubber, (meth)acrylonitrile-butadiene rubber, (meth)acrylic rubber, butyl rubber, fluororubber, a polyethylene oxide, polyvinylpyrrolidone, polyepichlorohydrin, polyphosphazene, poly(meth)acrylonitrile, an ethylene propylene diene monomer copolymer, polyvinylpyridine, chlorosulfonated polyethylene, latex, a polyester resin, a (meth)acrylic resin, a phenolic resin, an epoxy resin, polyvinyl alcohol, and a combination thereof.

When the aqueous binder is used as the negative electrode binder, a cellulose-based compound may be further contained to impart viscosity. This cellulose-based compound may be formed by mixing one or more of carboxymethyl cellulose, hydroxypropyl methyl cellulose, methyl cellulose, or an alkali metal salt of one of these. As the alkali metal, Na, K, or Li may be used.

The dry binder is a polymer material that may be fiberized, such as polytetrafluoroethylene, polyvinylidene fluoride, a polyvinylidene fluoride-hexafluoropropylene copolymer, polyethylene oxide, or a combination thereof.

The conductive material may be used to impart conductivity to the electrode. In a configured battery, any electronically conductive material that does not cause a chemical change may be used. Specific examples may include a carbon-based material such as natural graphite, artificial graphite, carbon black, acetylene black, Ketjenblack, carbon fibers, carbon nanofibers, carbon nanotubes; a metal-based material containing copper, nickel, aluminum, silver, etc. in the form of a metal powder or metal fiber; a conductive polymer such as a polyphenylene derivative; or a mixture thereof.

As the negative electrode current collector, one of a copper foil, a nickel foil, a stainless steel foil, a titanium foil, a nickel foam, a copper foam, a conductive metal-coated polymer substrate, and a combination thereof may be used.

**Electrolyte**

The electrolyte for the lithium secondary battery 100 may include a non-aqueous organic solvent and a lithium salt. The non-aqueous organic solvent may serve as a medium through which ions involved in the electrochemical reaction of the battery may move. The non-aqueous organic solvent may be a carbonate-based, an ester-based, ether-based, ketone-based, or alcohol-based solvent, an aprotic solvent, or a combination thereof.

The carbonate-based solvent may include dimethyl carbonate (DMC), diethyl carbonate (DEC), dipropyl carbonate (DPC), methylpropyl carbonate (MPC), ethylpropyl carbonate (EPC), methylethyl carbonate (MEC), ethylene carbonate (EC), propylene carbonate (PC), butylene carbonate (BC), and the like.

The ester-based solvent may include methyl acetate, ethyl acetate, n-propyl acetate, dimethyl acetate, methyl propionate, ethyl propionate, decanolide, mevalonolactone, valerolactone, caprolactone, and the like.

The ether-based solvent may include dibutyl ether, tetraglyme, diglyme, dimethoxyethane, 2-methyltetrahydrofuran, 2,5-dimethyltetrahydrofuran, tetrahydrofuran, and the like. In addition, the ketone-based solvent may include cyclohexanone, and the like. As the alcohol-based solvent, ethyl alcohol, isopropyl alcohol, and the like may be used, and as the aprotic solvent, a nitrile-based one such as R-CN (R being a straight, branched, or ring-shaped hydrocarbon group having 2 to 20 carbon atoms, and may include a double bond, an aromatic ring, or an ether group), an amide-based one such as dimethylformamide, a dioxolane-based such as 1,3-dioxolane or 1,4-dioxolane, a sulfolane-based one and the like may be used.

The non-aqueous organic solvent may be used alone or in a combination of two or more.

In addition, when using a carbonate-based solvent, a ring-shaped carbonate and a chain-shaped carbonate may be used by being mixed, and the ring-shaped carbonate and the chain-shaped carbonate may be mixed in a volume ratio of 1:1 to 1:9.

The lithium salt is a substance that may be dissolved in the organic solvent and may act as a source of lithium ions within the battery, enabling a basic operation of the lithium secondary battery and promoting the movement of lithium ions between the positive electrode and the negative electrode. Representative examples of the lithium salt may include one or more of $LiPF_6$, $LiBF_4$, $LiSbF_6$, $LiAsF_6$, $LiClO_4$, $LiAlO_2$, $LiAlCl_4$, $LiPO_2F_2$, LiCl, LiI, $LiN(SO_3C_2F_5)_2$, $Li(FSO_2)_2N$ (lithium bis(fluorosulfonyl)imide (LiFSI)), $LiC_4F_9SO_3$, $LiN(C_xF_{2x+1}SO_2)(C_yF_{2y+1}SO_2)$ (x and y being integers from 1 to 20), lithium trifluoromethanesulfonate, lithium tetrafluoroethanesulfonate, lithium difluorobis(oxalato)phosphate (LiDFOB), and lithium bis(oxalato)borate (LiBOB).

## Separator 30

Depending on the type of the lithium secondary battery 100, the separator 30 may be present between the positive electrode 10 and the negative electrode 20. As the separator 30, polyethylene, polypropylene, polyvinylidene fluoride, or a multilayered film of two or more layers thereof may be used, and a mixed multilayered film such as a polyethylene/polypropylene two-layer separator, a polyethylene/polypropylene/polyethylene three-layer separator, or a polypropylene/polyethylene/polypropylene three-layer separator may also be used as the separator 30.

The separator 30 may include a porous substrate and a coating layer containing an organic material, an inorganic material, or a combination thereof located on one surface or both surfaces of the porous substrate.

The porous substrate may be a polymer film formed of any one of a polymer, or a copolymer or mixture of two or more of these selected from polyolefins such as polyethylene or polypropylene, polyesters such as polyethylene terephthalate or polybutylene terephthalate, polyacetal, polyamide, polyimide, polycarbonate, polyether ketone, polyarylether ketone, polyetherimide, polyamideimide, polybenzimidazole, polyethersulfone, a polyphenylene oxide, a cyclic olefin copolymer, polyphenylene sulfide, polyethylene naphthalate, a glass fiber, Teflon, and polytetrafluoroethylene.

The organic material may include a polyvinylidene fluoride-based polymer or a (meth)acryl-based polymer.

The inorganic material may include inorganic particles selected from $Al_2O_3$, $SiO_2$, $TiO_2$, $SnO_2$, $CeO_2$, MgO, NiO, CaO, GaO, ZnO, $ZrO_2$, $Y_2O_3$, $SrTiO_3$, $BaTiO_3$, $Mg(OH)_2$, boehmite, and a combination thereof. But the present disclosure is not limited to these examples.

The organic material and the inorganic material may be present by being mixed in one coating layer or may be present in a form in which a coating layer containing an organic material and a coating layer containing an inorganic material are stacked.

As described in FIGS. 1 to 4, the secondary battery 100 according to an embodiment may include the electrode assembly 40 and the case 50 in which the electrode assembly 40 is accommodated. The electrode assembly 40 may include the positive electrode 10, the negative electrode 20, and the separator 30 located between the positive electrode 10 and the negative electrode 20. In addition, the secondary battery 100 may further include an electrolyte, which is embedded in the case 50 together with the electrode assembly 40 and impregnated in the electrode assembly 40.

Specifically, the electrode included in the electrode assembly 40 may be impregnated with the electrolyte. The electrode may include the positive electrode 10 and/or the negative electrode 20. The electrode may include a current collector and an active material layer formed on at least one surface of the current collector. The active material layer may include an active material, a binder, and/or a conductive material.

The electrolyte may be impregnated in the active material layer. Since the electrolyte is impregnated in the active material layer, ions included in the electrolyte may be electrically connected to the electrode. Therefore, the secondary battery may charge and/or discharge electrical energy.

As such, it may be necessary to evaluate the impregnation of the electrode indicating the degree to which the electrode is sufficiently impregnated with the electrolyte. This is because, based on the evaluated impregnation, appropriate impregnation conditions with improved impregnation and impregnation speed can be derived. **In** addition, it is possible to increase the efficiency of an impregnation process of the electrode by impregnating the electrode under the appropriate impregnation conditions. Furthermore, the secondary battery with increased charge-discharge efficiency can be provided by impregnating the electrode under the appropriate impregnation conditions.

Image analysis, gas amount measurement, and impedance measurement, etc. for the electrode can be used to determine whether an electrode is sufficiently impregnated. However, in these cases, a charge-discharge test of the secondary battery is required, which is less in efficient in terms of time and/or cost. Furthermore, using such methods, it is only possible to determine the presence or absence of impregnation, and it is difficult to evaluate the degree of impregnation of the electrode.

Therefore, a method for efficiently evaluating the impregnation of the electrode will be described below.

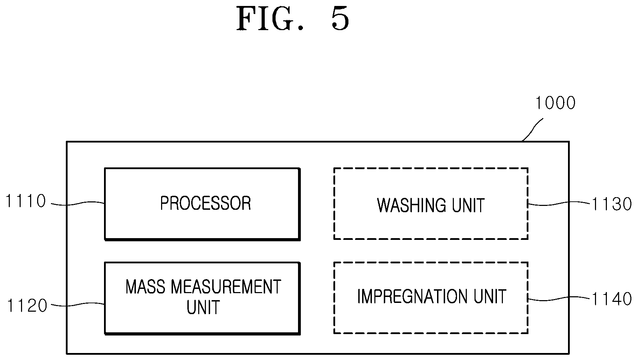

FIG. 5 is a block diagram schematically showing an impregnation evaluation device 100 according to one embodiment.

The impregnation evaluation device 1000 may evaluate the impregnation of the electrode plate such as the electrode described in FIGS. 1 to 4, including the positive electrode 10 and/or the negative electrode 20. In this case, the impregnation refers to a degree to which the electrolyte is impregnated into the electrode plate.

The impregnation evaluation device 1000 may determine whether the electrode plate is impregnated with the electrolyte. In addition, the impregnation evaluation device 1000 may evaluate a degree of the electrolyte impregnated into the electrode plate.

To this end, as shown in FIG. 5, the impregnation evaluation device 1000 may include a processor 1110 and a mass measurement unit 1120. The impregnation evaluation device 1000 may further include a washing unit 1130and an impregnation unit 1140. However, components included in the impregnation evaluation device 1000 are not limited to those shown in FIG. 5. That is, the impregnation evaluation device 1000 may include only some of the components shown in FIG. 5 and/or may further include additional components other than the components shown in FIG. 5. For example, the impregnation evaluation device 1000 may further include a memory for storing acquired data and/or commands, a communication unit for transmitting and receiving data with an external server, and/or an input/output unit for receiving the commands from a user and/or outputting the data to the user.

With the components, the impregnation evaluation device 1000 may calculate a theoretical mass, which is a mass of the electrode plate when the electrode plate is 100% impregnated with the electrolyte. The impregnation evaluation device 1000 may determine an actual mass, which is a mass of the electrode plate when the electrode plate is actually impregnated with the electrolyte. The impregnation evaluation device 1000 may evaluate the impregnation by comparing the theoretical mass with the actual mass and/or derive an impregnation time of the electrode plate. Each of the components of the impregnation evaluation device 1000 shown in FIG. 5 will be described in detail below.

The impregnation unit 1140 may impregnate the electrode plate. In an embodiment, the impregnation unit 1140 may allow the dried electrode plate to be immersed and impregnated in the electrolyte. For example, the impregnation unit 1140 may immerse a sample formed from the dried electrode plate in the electrolyte so that the sample may be impregnated with the electrolyte.

The washing unit 1130 may wash the electrode plate that is impregnated by the impregnation unit 1140. The washing unit 1130 may include, for example, a micropipette. For example, the washing unit 1130 may wash the impregnated electrode plate by spraying a washing solvent onto the impregnated electrode plate through the micropipette. In this case, the washing solvent may include a DMC solvent, but the present disclosure is not limited thereto. As an example, the amount may be, for example, 1 ml or less. When the amount exceeds 1 ml, there is a risk that the impregnated electrolyte may be washed away from the impregnated electrode plate and/or the electrode plate itself may be damaged. Therefore, a properly functioning washing unit 1130 can increase accuracy of a mass of the electrode plate calculated by the processor 1110.

FIG. 5 shows that the impregnation evaluation device 1000 according to one embodiment includes the impregnation unit 1140 and/or the washing unit 1130. However, the roles of the impregnation unit 1140 and/or the washing unit 1130 may be performed by an external device or a user.

The processor 1110 may calculate the theoretical mass of the electrode plate. As noted above, the theoretical mass of the electrode plate may be a theoretical mass of the electrode plate assuming that the electrode plate is 100% impregnated with the electrolyte. For example, the processor 1110 may calculate an increase in a mass of the electrode plate if the electrode plate were 100% impregnated with the electrolyte. Therefore, the processor 1110 may calculate the theoretical mass of the electrode plate. In embodiments, the processor 1110 may calculate the theoretical mass of the electrode plate for each impregnation time.

The mass measurement unit 1120 may measure the actual mass of the electrode plate. as noted above, the actual mass of the electrode plate is a mass of the electrode plate when the electrode plate is impregnated with the electrolyte. For example, the mass measurement unit 1120 may measure the actual mass of the electrode plate after impregnated by the impregnation unit 1140. In embodiments, the mass measurement unit 1120 may measure the actual mass of the electrode plate for each impregnation time.

The processor 1110 may evaluate the impregnation of the electrode plate by comparing the theoretical mass with the actual mass. In embodiments, the processor 1110 may compare the theoretical mass with the actual mass of the electrode plate for each impregnation time. The processor 1110 may determine a ratio of the actual mass of the electrode plate and the theoretical mass of the electrode plate. Therefore, the processor 1110 may evaluate the impregnation of the electrode plate. In addition, the processor 1110 may determine an impregnation time of a section where it is determined that the impregnation of the electrode plate is most appropriate. The processor 1110 may provide the corresponding impregnation time as an impregnation condition for the electrode plate.

With such a configuration, the impregnation evaluation device 1000 according to one embodiment can evaluate the impregnation of the electrode plate without a charge-discharge test on the secondary battery including the electrode plate. Further, the impregnation evaluation device 1000 can provide the appropriate impregnation conditions. Accordingly, the impregnation evaluation device 1000 according to an embodiment can reduce the cost and/or time required for evaluating the impregnation of the electrode plate and/or for the impregnation process.

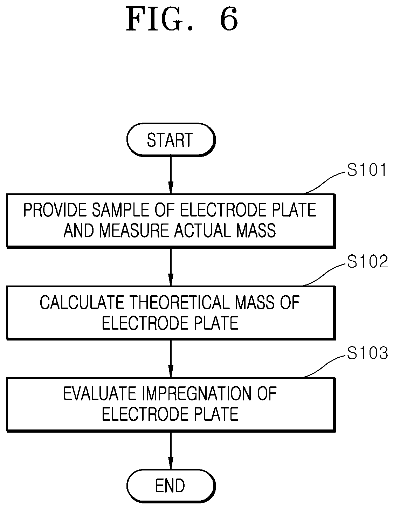

FIG. 6 is a flowchart showing an impregnation evaluation method according to an embodiment.

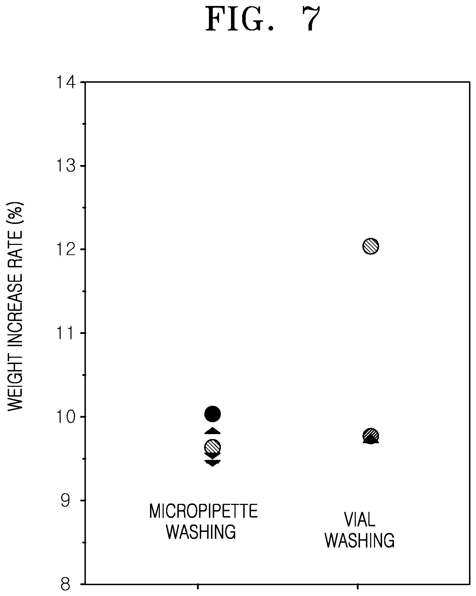

FIG. 7 is a graph for describing a washing unit according to an embodiment.

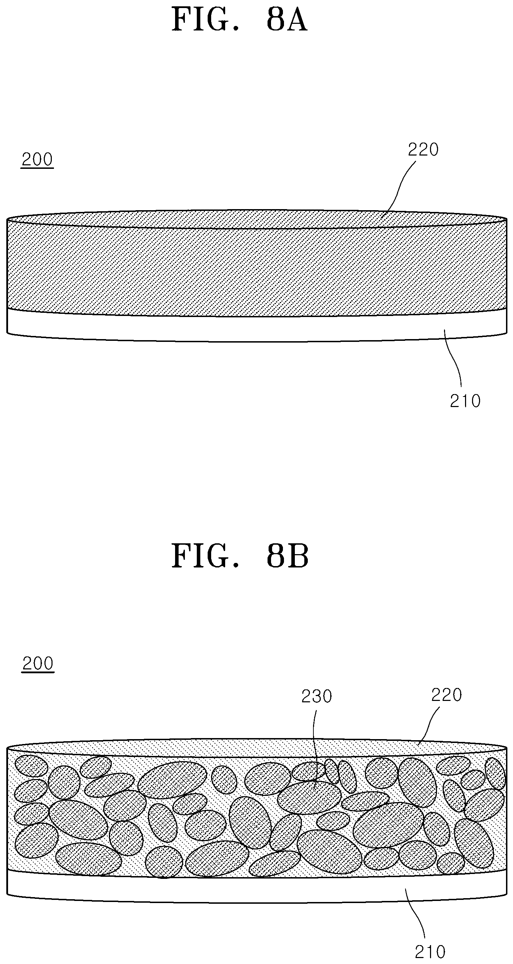

FIGS. 8A and 8B show an ideal electrode plate without pores and an actual electrode plate with pores.

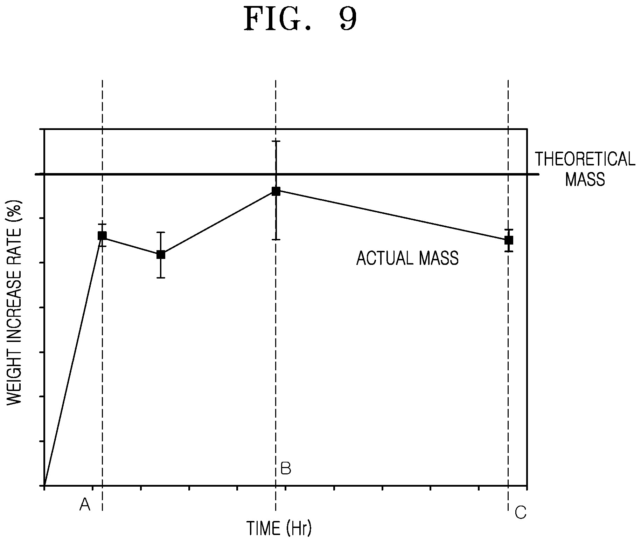

FIG. 9 is a graph of a theoretical mass versus an actual mass shown according to one embodiment.

Referring to FIG. 6, a method of evaluating electrolyte impregnation of the electrode plate using the impregnation evaluation device 1000 according to the embodiment shown in FIG. 5 will be described. However, the impregnation evaluation method described in FIG. 6 may be performed by devices other than the impregnation evaluation device 1000 according to other embodiments. The impregnation evaluation method described in FIG. 6 may also be performed by one or more devices or systems capable of performing each operation.

As shown in FIG. 6, the impregnation evaluation method according to an embodiment may include providing a sample of an electrode plate and measuring an actual mass thereof (S 101). To evaluate the impregnation of the electrode plate, the sample subject to evaluation is required. The sample may be obtained from the electrode plate subject to evaluation.

In this case, the electrode plate subject to evaluation may be any electrode plate. The electrode plate may include all forms of electrode plates in various shapes such as a coin cell, a stack cell, and a jelly roll. The electrode plate may be of any form with the electrode plate being "washed" (as will be described below). For the convenience of description, an example in which the electrode plate is applied to a secondary battery in a coin cell form will be described below.

The sample may be formed by punching the electrode plate into a size and shape suitable for use of a coin cell. For example, the electrode plate applicable to a coin cell may have a diameter of 18 pi and a circular cross-section. In this case, the sample may be formed by punching the electrode plate into a circular shape of 18-pi. For convenience of description, the sample manufactured in this way will be referred to hereinafter as the electrode plate.

The electrode plate may be dried to remove moisture. For example, the electrode plate may be dried naturally or in an oven such that the electrode plate is dried for several hours or several days. As a specific example, the electrode plate may be dried in a convection oven at 120 degrees or higher for one day or longer.

The dried electrode plate may be impregnated with the electrolyte. For example, the impregnation unit 1140 may immerse the electrode plate in the electrolyte using a vial in a moisture-controlled dry room environment. Therefore, the impregnation unit 1140 may impregnate the electrode plate for a predetermined time.

The impregnated electrode plate may be washed. For example, the washing unit 1130 may wash the electrolyte away from the electrode plate using a DMC solvent. In this case, as described in FIG. 5, the washing unit 1130 may include, for example, a micropipette, and may wash the electrode plate using the micropipette. FIG. 7 shows a weight (mass) increase rate for a case where the washing unit 1130 washes the electrode plate using the micropipette and a case where the washing unit 1130 washes the electrode plate using the vial. Each of the solid dots, the stripped dots and the triangle dots in FIG. 7 represents one electrode plate. The weight increase rate refers to a mass of the electrode plate after impregnation and washing compared to a mass of the electrode plate before impregnation. It can be seen in FIG. 7 that the weight increase rate has a spread of 0.5% range for each electrode plate when the electrode plate is washed using the micropipette. However, it can also be seen in FIG. 7 that the weight increase rate has a spread of 2.5% range for each electrode plate when the electrode plate is washed using the vial, which is wider spread than the case the electrode plate is washed using the micropipette. Through this, when the electrode plate is washed by the micropipette rather than the vial, it can be seen that the "theoretical" value and "real" value are more in agreement. Therefore, it is preferable that the washing unit 1130 include the micropipette for accurate mass measurement of the impregnated electrode plate. When the electrode plate is manufactured in the form of a stacked cell or a jelly roll rather than a coin cell, the washing unit 1130 may wash a surface of the electrode plate by unrolling or disassembling the electrode plate.

In addition, the washing unit 1130 may blow the remaining solvent off using a blower after the washing of the electrode plate is finished. Through this, the washing unit 1130 may provide for the actual mass of the electrode plate to be accurately measured in a later operation.

The washed electrode plate may be dried. For example, the electrode plate may be vacuum dried at room temperature for one day or longer. However, the electrode plate may also be dried in an oven in a shorter time. The oven may volatilize the electrolyte from the surface of the electrode plate through a method such as electric resistance heating, infrared heating, or microwave heating.

The mass measurement unit 1120 may measure the mass of the washed and dried electrode plate. That is, the mass measurement unit 1120 may determine the actual mass of the electrode plate.

As shown in FIG. 6, the impregnation evaluation method according to one embodiment includes calculating the theoretical mass of the electrode plate (S102). The processor 1110 may calculate the theoretical mass of the electrode plate.

An electrode plate 200 is shown in FIG. 8. The electrode plate 200 may include a current collector 210 and an active material layer 220 provided on at least a portion of at least one surface of the current collector 210. The active material layer 220 may include an active material, a binder, and/or a conductive material. A material of each component included in the electrode plate 200 may be the same as or similar to the material described in FIGS. 1 to 4.

FIG. 8A shows an ideal electrode plate. The ideal electrode plate may be, for example, an electrode plate in which no pores are formed in the active material layer 220. FIG. 8B shows an actual electrode plate. The actual electrode plate may an electrode plate have pores 230 formed in the active material layer 220. In this case, the actual electrode plate may have a greater volume for impregnation than the ideal electrode plate. Thus, the actual electrode plate may be impregnated with more impregnating liquid compared to the ideal electrode plate.

As shown in FIG. 8B, the electrode plate 200 may include pores 230 in the active material layer 220. Accordingly, to calculate the theoretical mass of the electrode plate 200, the pores 230 formed in the electrode plate 200 should be considered. This is because a degree of the impregnating solution included in the electrode plate 200 may be changed by the pores 230.

The processor 1110 may calculate the theoretical mass of the electrode plate 200 using, for example, the following method. However, the method described below is merely an example. The method for calculating the theoretical mass of the electrode plate 200 may include other methods capable of obtaining the mass when the electrode plate 200 is 100% impregnated.

The processor 1110 may calculate a theoretical mass m of the electrode plate using the following:

[Equation 1]

$$m = m_a + m_b + m_c$$

(1) In Equation 1, m denotes the theoretical mass of the electrode plate.
(2) In Equation 1, $m_a$ denotes the mass of the electrode plate before impregnation. In this case, $m_a$ denotes, for example, a value measured by the mass measurement unit 1120 before the electrode plate 200 is impregnated.
(3) In Equation 1, $m_b$ denotes a mass of the electrolyte impregnated in the active material layer 220 of the electrode plate after the electrode plate is impregnated. In this case, $m_b$ may be calculated by the processor 1110 according to the following:

[Equation 2]

$$m_b = d_{EC} \cdot V_v$$

In Equation 2, $d_{EC}$ denotes a density of the active material layer 220 of the electrode plate. The density ($d_{EC}$) of the active material layer 220 of the electrode plate is a value obtained by dividing a mass of the active material layer 220 of the electrode plate by a volume thereof.

In Equation 2, $V_v$ denotes a volume of the pore 230 formed in the electrode plate. The processor 1110 may calculate the volume ($V_v$) of the pore using the following:

[Equation 3]

$$V_v = V_{EC} - V_a$$

In Equation 3, a volume ($V_{EC}$) of the active material layer 220 of the electrode plate includes the volume ($V_a$) of the active material of the electrode plate and a volume ($V_v$) of the pores formed in the electrode plate. Therefore, to calculate the volume ($V_v$) of the pores, the processor 1110 uses the difference between volumes thereof.

In Equation 3, $V_{EC}$ denotes the volume of the active material layer 220 of the electrode plate. For example, when the electrode plate is used in a coin cell and has a circular cross-section, the processor 1110 may calculate the volume ($V_{EC}$) of the active material layer 220 of the electrode plate by multiplying a height of the active material layer 220 by a cross-sectional area of the electrode plate 200. And the processor 1110 may calculate the cross-sectional area of the electrode plate 200 by multiplying $\pi$ by the square of a radius of the electrode plate 200. In this case, the processor 1110 may calculate the height of the active material layer 220 by subtracting a height of the current collector 210 from a total height of the electrode plate 200.

In Equation 3, $V_a$ denotes the volume of the active material of the electrode plate. In Equation 3, for the convenience of description, a value obtained by subtracting $V_a$ from $V_{EC}$ is referred to as $V_v$. Therefore, $V_a$ denotes the sum of the volumes of components included in the active material layer, such as the active material, the conductive material, and the binder. In general, since the volume of the active material in the volume ($V_{EC}$) of the active material layer is enough that the volumes of the conductive material and/or the binder may be ignored, $V_a$ denotes only the volume of the active material.

(4) In Equation 1, $m_c$ denotes the mass of the electrolyte included in the pore 230 formed in the electrode plate after the electrode plate is impregnated. In this case, $m_c$ may be calculated by the processor 1110 according to the following:

[Equation 4]

$$m_c = M_e \cdot n_e$$

In Equation 4, $M_e$ denotes a molar mass of the electrolyte with which the electrode plate 200 is impregnated.

In Equation 4, $n_e$ denotes the number of moles of the electrolyte with which the electrode plate 200 is impregnated. In this case, the number of moles of the electrolyte ($n_e$) is a value obtained by applying the volume of the pore 230 formed in the electrode plate 200.

Through this method, the processor 1110 may calculate the theoretical mass of the electrode plate 200. The methods described in S102 are explained as an example of a case where the electrode plate is applied to the coin cell. But when the electrode plate is applied to a secondary battery in a form other than a coin cell, the processor 1110 may calculate values such as a volume, radius, height, and density of the electrode plate according to each shape of the electrode plate.

Meanwhile, the impregnation evaluation device 1000 may measure the actual mass of the electrode plate through the mass measurement unit 1120 after calculating the theoretical mass of the electrode plate through the processor 1110. Alternatively, the impregnation evaluation device 1000 may simultaneously measure the actual mass of the electrode plate through the mass measurement unit 1120 while calculating the theoretical mass of the electrode plate through the processor 1110. That is, an order in which the impregnation evaluation device 1000 measures the theoretical and actual masses of the electrode plate is not limited in the present disclosure.

As shown in FIG. 6, the impregnation evaluation method according to an embodiment may include evaluating the impregnation of the electrode plate (S103).

The processor 1110 may evaluate the impregnation of the electrode plate based on the theoretical mass and actual mass of the electrode plate. In addition, the processor 1110 may derive the most appropriate impregnation conditions for the impregnation of the electrode plate based on the evaluation. To derive the appropriate impregnation conditions, the processor 1110 may determine a weight increase of the electrode plate after impregnation for each impregnation time.

For example, the processor 1110 may determine the actual mass value compared to the theoretical mass value. In this case, when the actual mass value is closer to the theoretical mass value, the processor 1110 determines that the corresponding electrode plate has been sufficiently impregnated. That is, the processor 1110 may determine that a degree of the impregnation of the electrode plate is sufficient and the impregnation is excellent. In addition, when there is a significant difference between the actual mass and theoretical mass values, the processor 1110 determines that the electrode plate has not been sufficiently impregnated. That is, the processor 1110 determines that the degree of the impregnation of the electrode plate is insufficient and the impregnation is poor. For example, when the percentage of the actual mass compared to the theoretical mass is 100%, the processor 1110 determines that the degree of the impregnation of the electrode plate is 100%. As another example, when the percentage of the actual mass to the theoretical mass is 80%, the processor 1110 determines that the degree of the impregnation of the corresponding electrode plate is 80%.

In addition, the processor 1110 may determine the degree of the impregnation of the electrode plate for each amount of impregnation time. For example, the processor 1110 may determine the degree of the impregnation of the electrode plate on an hour-by-hour basis for 1 to 24 hours. The processor 1110 may determine the degree of the impregnation comparing the theoretical mass with the actual mass of the electrode plate for each impregnation time. Therefore, the processor 1110 may determine the appropriate impregnation conditions based on the required degree of the impregnation and the required impregnation time. That is, the processor 1110 may determine the ratio of the actual mass compared to the theoretical mass for each impregnation time and determine that an impregnation time that meets a predetermined ratio among ratios determined for each impregnation time is the most appropriate impregnation condition. In this case, the predetermined ratio may vary depending on specifications required for the corresponding electrode plate or a field to which the electrode plate is applied.

FIG. 9 is a graph showing the determination made by the processor 1110. In particular, FIG. 9 shows a graph created based on an experiment on a weight increase rate of the actual mass as compared to the theoretical mass. In FIG. 9, the x-axis represents the time (Hr, hours) for which the electrode plate is impregnated with the electrolyte. The y-axis represents the actual mass of the electrode plate as a percentage of the theoretical mass, thus representing the weight increase rate (%). In FIG. 9, the bold line drawn parallel to the time axis represents the theoretical mass. In addition, the values drawn perpendicular to the time axis represent an error range as error bars for the actual mass. In an example, the electrode plate may be required to be impregnated at a faster impregnation speed. In this case, based on the graph in FIG. 9, the processor 1110 may determine that impregnating the electrode plate for time A is the most appropriate condition for impregnating the electrode plate. In another example, the degree of the impregnation of the electrode plate may need to be high. In this case, based on the graph in FIG. 9, the processor 1110 may determine that impregnating the electrode plate for time B is the most appropriate condition for impregnating the electrode plate. Meanwhile, based on a result value when the impregnation time is C, the processor 1110 may determine that when the electrode plate is impregnated for longer than time B, an amount of the impregnation of the electrode plate is actually reduced. In this case, the processor 1110 may determine that impregnating the electrode plate for longer than time B is not a preferable impregnation condition.

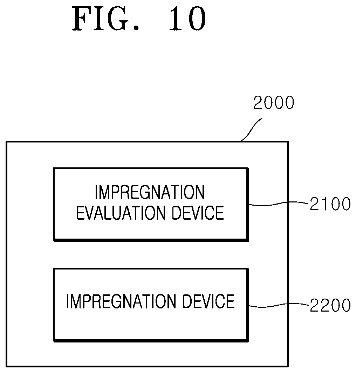

FIG. 10 is a block diagram schematically showing a secondary battery production system according to one embodiment. The secondary battery production system 2000 may impregnate the electrode plate under the most efficient conditions using the impregnation evaluation method described in FIGS. 5 to 9. The secondary battery production system 2000 may manufacture the secondary battery using the impregnated electrode plate.

To this end, the secondary battery production system 2000 may include an impregnation evaluation device 2100 and an impregnation device 2200. However, components included in the secondary battery production system 2000 are not limited to those shown in FIG. 10. That is, the secondary battery production system 2000 may include only some of the components shown in FIG. 10 and/or may further include additional components other than the components shown in FIG. 10. For example, the secondary battery production system 2000 may further include a cap assembly unit for sealing the secondary battery 100 including the impregnated electrode plate and a formation unit for testing charge-discharge of the secondary battery by performing the charge-discharge of the secondary battery.

The impregnation evaluation device 2100 may be the same as or similar to the impregnation evaluation device 1000 described with reference to FIGS. 5 to 9. As described above, the impregnation evaluation device 2100 may evaluate the impregnation for the sample of the electrode plate.

The impregnation device 2200 may impregnate the electrode plate. For example, the impregnation device 2200 may impregnate the electrode plate based on the result evaluated for the sample of the electrode plate by the impregnation evaluation device 2100. In some embodiments, the impregnation device 2200 may immerse the electrode plate in the electrolyte to impregnate the electrode plate with the electrolyte. To this end, the impregnation device 2200 may include an electrolyte injection unit for injecting the electrolyte. The electrolyte injected by the impregnation device 2200 may be the same as or similar to the electrolyte described with reference to FIGS. 1 to 4. The impregnation device 2200 also may impregnate the electrode plate for the optimal impregnation time derived by the impregnation evaluation device 2100.

The amount of impregnation required for each secondary battery may vary. This is because specifications applied to the secondary batteries may be different or capacities of the secondary batteries may be different. The secondary battery production system 2000 may allow the electrode plate to be sufficiently impregnated with the electrolyte in accordance with each of these requirements. Therefore, the secondary battery production system 2000 can provide the secondary battery with an excellent ability to charge and discharge electrical energy. In addition, the secondary battery production system 2000 may impregnate the electrode plate for the most appropriate time required for impregnation. Therefore, the secondary battery production system 2000 can reduce the time for impregnating the electrode plate. With this configuration, the secondary battery production system 2000 can efficiently manufacture the secondary battery.

According to the present disclosure, the impregnation of an electrode can be determined without charging and discharging a secondary battery including the electrode.

In addition, according to the present disclosure, the impregnation of each of a negative electrode and a positive electrode can be determined.

According to the present disclosure, the electrode can be provided by measuring impregnation and an impregnation speed of the electrode and/or determining an optimal impregnation environment for the electrode.

According to the present disclosure, the cost and/or time for securing impregnation conditions for the electrode can be reduced.

According to the present disclosure, it is possible to efficiently impregnate the electrode and/or manufacture the secondary battery including the impregnated electrode.

However, the effects that can be achieved through the present disclosure are not limited to the effects expressly described herein, and other technical effects not mentioned will be clearly understood by those skilled in the art from the disclosure.

Although the present disclosure has been described above with limited examples and drawings, the present disclosure is not limited thereto, and various modifications and variations may be made by those skilled in the art in the technical field to which the present disclosure belongs within the technical scope of the present disclosure.

**Claims**

1. An impregnation evaluation device (1000, 2100) comprising:

   a processor (1110) configured to calculate a theoretical mass of an electrode plate; and
   a mass measuring unit configured to measure an actual mass of the electrode plate,
   wherein the processor (1110) is configured evaluate impregnation of the electrode plate by comparing the theoretical mass to the actual mass.

2. The impregnation evaluation device (1000, 2100) of claim 1, wherein the theoretical mass of the electrode plate is a mass when the electrode plate is 100% impregnated with an electrolyte.

3. The impregnation evaluation device (1000, 2100) of claim 1 or 2, wherein the processor (1110) is configured to calculate the theoretical mass of the electrode plate using Equation 1:

   [Equation 1]

   $$m = m_a + m_b + m_c,$$

   in which m denotes the theoretical mass of the electrode plate, $m_a$ denotes a mass before the electrode plate is impregnated, $m_b$ denotes a mass of the electrolyte impregnated in an active material layer (220) of the electrode plate after the electrode plate is impregnated, and $m_c$ denotes a mass of the electrolyte included in pores (230) formed in the electrode plate after the electrode plate is impregnated.

4. The impregnation evaluation device (1000, 2100) of claim 3, wherein the processor (1110) is configured to calculate $m_b$ using Equation 2:

   [Equation 2]

   $$m_b = d_{EC} \cdot V_v,$$

   in which $d_{EC}$ denotes a density of the active material layer of the electrode plate, and $V_v$ denotes a volume of the pores (230) formed in the electrode plate.

5. The impregnation evaluation device (1000, 2100) of claim 4, wherein the processor (1110) is configured to calculate $V_v$ using Equation 3:

   [Equation 3]

   $$V_v = V_{EC} - V_a,$$

   in which $V_{EC}$ denotes a volume of the active material layer of the electrode plate, and $V_a$ denotes a volume of an active material included in the active material layer of the electrode plate.

6. The impregnation evaluation device (1000, 2100) according to any one of the claims 3 to 5, wherein the processor (1110) is configured to calculate $m_c$ using Equation 4:

[Equation 4]

$$m_c = M_e \cdot n_e,$$

in which $M_e$ denotes a molar mass of the electrolyte solution, and $n_e$ denotes the number of moles of the electrolyte.

7. The impregnation evaluation device (1000, 2100) according to any one of claims 1 to 6, further comprising:

an impregnation unit (1140) configured to impregnate the electrode plate; and
a washing unit (1130) configured to wash the impregnated electrode plate,
wherein the mass measuring unit is configured to measure the actual mass of the electrode plate washed by the washing unit (1130).

8. The impregnation evaluation device (1000, 2100) of claim 7, wherein the washing unit (1130) includes a micropipette.

9. The impregnation evaluation device (1000, 2100) according to any one of claims 1 to 8, wherein the processor (1110) is configured to determine that the impregnation of the electrode plate as a ratio of the actual mass compared to the theoretical mass is higher.

10. The impregnation evaluation device (1000, 2100) according to any one of claims 1 to 9, wherein the processor (1110) is configured to determine a ratio of the actual mass compared to the theoretical mass for impregnation times, and the processor (1110) is configured to determine an impregnation time that meets a predetermined ratio among ratios for each impregnation time as an impregnation condition.

11. A secondary battery production system comprising:

an impregnation evaluation device (1000, 2100) configured to evaluate impregnation of an electrode plate by comparing a theoretical mass of the electrode plate to an actual mass of the electrode plate; and
an impregnation device configured to impregnate the electrode plate based on an evaluation result by the impregnation evaluation device (1000, 2100).

12. The secondary battery production system of claim 11, wherein the theoretical mass of the electrode plate is a mass when the electrode plate is 100% impregnated with an electrolyte.

13. The secondary battery production system of claim 11 or 12, wherein the impregnation evaluation device (1000, 2100) is configured to calculate the theoretical mass of the electrode plate using Equation 1:

[Equation 1]

$$m = m_a + m_b + m_c,$$

in which m denotes the theoretical mass of the electrode plate, $m_a$ denotes a mass before the electrode plate is impregnated, $m_b$ denotes a mass of the electrolyte impregnated in an active material layer of the electrode plate after the electrode plate is impregnated, and $m_c$ denotes a mass of the electrolyte included in pores (230) formed in the electrode plate after the electrode plate is impregnated.

14. The secondary battery production system according to any one of claims 11 to 13, wherein the impregnation evaluation device (1000, 2100) is configured to measure the actual mass by impregnating the electrode plate, washing the impregnated electrode plate, and measuring a mass of the washed electrode plate.

15. The secondary battery production system according to any one of claims 11 to 14, wherein the impregnation evaluation device (1000, 2100) is configured to determine a ratio of the actual mass compared to the theoretical mass for impregnation times and determines an impregnation condition for the electrode plate.

# FIG. 1

# FIG. 2

# FIG. 3

# FIG. 4

# FIG. 5

1000

1110 PROCESSOR

1120 MASS MEASUREMENT UNIT

WASHING UNIT 1130

IMPREGNATION UNIT 1140

# FIG. 6

```
        ( START )

              |
              ▼                                    ⸺S101
  ┌──────────────────────────────────┐
  │   PROVIDE SAMPLE OF ELECTRODE PLATE   │
  │      AND MEASURE ACTUAL MASS      │
  └──────────────────────────────────┘
              |
              ▼                                    ⸺S102
  ┌──────────────────────────────────┐
  │   CALCULATE THEORETICAL MASS OF   │
  │         ELECTRODE PLATE          │
  └──────────────────────────────────┘
              |
              ▼                                    ⸺S103
  ┌──────────────────────────────────┐
  │    EVALUATE IMPREGNATION OF       │
  │         ELECTRODE PLATE          │
  └──────────────────────────────────┘
              |
              ▼
        (  END  )
```

# FIG. 7

# FIG. 8A

200

220

210

# FIG. 8B

200

230

220

210

# FIG. 9

# FIG. 10